# EUROPEAN PATENT APPLICATION

(11) **EP 1 816 473 A1**
(43) Date of publication of application: **08.08.2007**
(21) Application number: 04821451.4
(22) Date of filing: 24.11.2004
(51) Int. Cl.: G01N 33/53

(54) **METHOD FOR QUANTITATIVELY DETECTING BIOLOGICAL TOXINS**

(71) Applicant: INSTITUT MOLEKULYARNOI BIOLOGII IM. V.A.ENGELGARDTA ROSSIISKOI AKADEMII NAUK, Moscow, 117984 (RU); INSTITUT BIOORGANICHESKOI KHIMII IMENI M.M. SHEMYA, Moscow, 117871 (RU)
(72) Inventor: DEMENTIEVA, Ekaterina Igorevna, Moscow, 117393 (RU); DJUKOVA, Veronika Igorevna, Moscow, 121165 (RU); ZASEDATELEV, Alexandr Sergeevich, Moscow, 117418 (RU); RUBINA, Alla Jurievna, Moscow, 117313 (RU); STOMAKHIN, Andrei Alexandrovich, Moscow, 117461 (RU); NESMEYANOV, Vladimir Andreevich, Moscow, 117437 (RU); GRISHIN, Evgeny Vasilievich, Moscow, 125284 (RU)
(74) Representative: Muir, Benjamin M. J.
(86) International application number: PCT/RU2004/000464
(87) International publication number: WO 2006/062427

(57) **Abstract**

The invention relates to analytical chemistry and to quantitative immunochemical analysis, in particular, to a method for immunochemical quantitative detection of various biological toxins by using biological microchips. A biological microchip comprises an ordered array of three-dimensional hydrogel cells on a solid support, which are produced by a method of photo- or chemically induced polymerization and contain immobilized antibodies to various bacterial, plant and animal biotoxins, or biotoxins. The use of microchips makes it possible to analyze a sample simultaneously for the presence of several biotoxins. The proposed method for detecting biotoxins can be used in medicine, in food industry, and in environmental protection.

## Description

### Field of the Art

The present invention relates to analytical biochemistry and to quantitative immunochemical analysis, and is concerned with quantitative detection of different biological toxins by an immunochemical method using three-dimensional hydrogel-based microchips.

The proposed method of detecting biotoxins can be used in medicine, in food industry, in environmental protection. At the present time the development of rapid and sensitive methods of analysis of biological toxins becomes of importance in connection with a threat of bioterrorism, because many of natural toxins may be used as biological weapon components.

### State of the Art

At the present time numerous bacterial toxins, phytotoxins and zootoxins are known, which have strong toxic effect on human organism. The strongest toxins produced by microorganisms are botulinus, tetanus and cholera toxins; the strongest phytotoxins are ricin and abrin. There also exist a large number of toxins secreted by poisonous animals: snakes, spiders, scorpions, etc. Most biotoxins are of polypeptide nature, but low-molecular compounds having a high toxicity are also known, for example, tetrodotoxin (blowfishes), T-2 toxin (fungi), blue-green algae toxins.

At the present time, for the identification and analysis of both toxins and organisms producing them, various laboratory methods are employed, comprising tests on animals, microbiological methods, DNA analysis with the use of PCR, immunological methods: direct and indirect radioimmunological, immunofluorescent and immunoenzymatic assay. The most sensitive, rapid and convenient method for analyzing toxins is an immunochemical method with the use of antibodies against toxins. The sensitivity of the immunoassay of toxins with the use of a classical "plate" variant reaches 0.01 to 1 ng of substance in 1 ml of solution under investigation (for instance, in the case of ricin [1], staphylococcus enterotoxin B [2], diphtheria toxin [3]).
[1] M.A. Poli, V.R. Rivera, J.F., Hevetson, G.A. Merril, Detection of ricin by colorimetric and chemiluminescence ELISA, Toxicon, 1994, 32, 1371-1377.
[2] M.A. Poli, V.R. Rivera,, D. Neal, Sensitive and specific colorimetric ELISAs for Staphylococcus aureus enterotoxins A and B in urine and buffer, Toxicon, 2002, 38, 1723-1726.
[3] K.H. Engler, A. Efstratiou, Rapid enzyme immunoassay for determination of toxi-genicity among clinical isolates of corynebacteria, J. Clin. Microbiol., 2004, 38, 1385-1389.
   Conventional immunological methods do not allow carrying out a simultaneous test for the presence of several toxins in a sample. Therefore there is a need for developing a rapid, sensitive and effective method of simultaneous parallel analysis. A parallel analysis of samples for the presence of several compounds is achieved with the use of microchips - arrays of individual cells containing various probes (proteins, receptors, antibodies, antigens, etc.). Carrying out simultaneous analysis of a sample for many parameters significantly increases the effectiveness of the analysis, makes it possible to miniaturize carrying out of investigations, and appreciably reduces the amount of material to be investigated.
   The use of chips of different construction for detecting biological toxins has been described. A biosensor chip based on immobilized antibodies against biotoxins was proposed for simultaneous immunological detection of biological toxins [4].
[4] F.S. Ligler, C.R. Taitt, L.S. Shriver-Lake, K.E. Sapsford, Y. Shubin, J.P. Golden, Array biosensor for detection of toxins, Anal. Bioanal. Chem., 2003, 377, 469-477.
   The biochip consisted of two elements (blocks). Channels with immobilized antibodies were arranged on a slide plate. Immobilization was carried out by forming an avidin-biotinylated antibodies complex. Toxins of protein nature were analyzed with the aid of sandwich analysis, using a pair of corresponding antibodies. Solutions of antigens and fluorescence-labeled developing antibodies were supplied to the immobilized antibodies through channels on the second block oriented perpendicular to columns of the immobilized antibodies. Fluorescent signals of antibody-antigen-labeled antibody complexes were registered with the aid of a confocal microscope. Toxin detection limits were 1.6 ng/ml for cholera toxin, 8 ng/ml for ricin, 40 ng/ml for botulinus toxin A, 4 ng/ml for staphylococcal enterotoxin B, 6.2·10⁴ CFU/ml for *Bacillus globigii* bacteria. This biosensor was also used for detecting low-molecular toxins by competitive immunoassay.
   For direct immunoassay of fluorescein-labeled staphylococcus and cholera toxins, Nanogen Company (USA) proposed electronic microchips with immobilized antibodies [5].
[5] K.L. Ewalt, R.W. Haigis, R. Rooney, D. Ackley, M. Krihak, Detection of biological toxins on an active electronic microchip, Anal. Biochem., 2001, 289, 162-172.
   Immobilization of biotinylated antibodies on a microchip was performed by forming avidin-biotin complexes on avidin-containing microsites. Microsites were supplied with electrodes, biotinylated antibodies and solutions of toxins, and solutions of toxins to be analyzed were fed thereto under the effect of electric field. In that case experiments with analytes were carried out only with fluorescently labeled toxins.
   Biopraxis Company (USA) is developing a microchip technology in which Raman spectroscopy (Raman scattering) is employed for signal detection [6].
[6] A.E. Grow, L.L. Wood, J.L. Claycomb, P.A. Thompson, New biochip technology for label-free detection of pathogens and their toxins, J. Microbiol. Methods, 2003, 53, 221-233.
   Biomolecules, in particular, antibodies against biotoxins, are immobilized on a metallic surface in loci having a diameter of about 1 micron. After treating the microchip with an analyte-containing solution, formation of an antibody-antigen complex takes place. This formation is detected by obtaining Raman scattering spectra from which the spectra of free antibodies are subtracted. The possibility of detecting B (1) and G (1) afla-toxins from a mixture has been demonstrated.
   Three-dimensional microchips based on polyacrylamide hydrogels were developed for the first time at IMB RAS [7].
[7] G.M. Ershov, A.D. Mirzabekov, Method of manufacturing a matrix for the detection of mismatches, US Patent No. 5770721.
   At the present time gel microchips are produced by copolymerization and polymerization immobilization methods [8, 9].
[8] A.D. Mirzabekov, A.Yu. Rubina, S.V. Pan'kov, B.K. Chernov, Method of immobilization of oligonucleotides containing unsaturated groups in polymeric hydrogels in the formation of microchip. Russian Patent No. 2175972, November 20, 2001 (**Bulletin of Inventions**, 2001, No. 25).
[9] A.D. Mirzabekov, A.Yu. Rubina, S.V. Pan'kov, Method of polymerization immobilization of biological macromolecules and composition for effecting same, RF Patent No. 2216547.
   The technology of producing hydrogel microchips comprises the steps of preparation of a support (glass, plastic, silicon) (1), applying a polymerization mixture containing gel components and substances to be immobilized, in the form of drops to a substrate with the help of a robot (2), photo-induced polymerization of a gel to form gel elements containing immobilized probes (3). As a result, arrays of discrete gel elements are obtained, each of which contains an immobilized probe. Oligonucleotides, DNAs, proteins, various low-molecular compounds can be used as probes [10-12]. It has been shown that protein microchips manufactured by the copolymerization method can be used for the investigation of protein-protein interactions, particularly, antigen-antibody interactions, for carrying out immunochemical and enzymatic reactions [11, 12].
[10] A.Yu. Rubina, S.V. Pan'kov, E.I. Dementieva, D.N. Pen'kov, A.V. Butygin, V.A. Vasiliskov, A.V. Chudinov, A.L. Mikheikin, V.M. Mikhailovich, A.D. Mirzabekov, Hydrogel drop microchips with immobilized DNA: properties and methods for large-scale production, Anal. Biochem., 2004, 325, 92-106.
[11] A.Yu. Rubina, S.V. Pan'kov, S.M. Ivanov, E.I. Dementieva, A.D. Mirzabekov, Protein microchips, Dotel. Biochem. Biopbys, 2001 381, 419-422 (Eng. Transl.)
[12] A.Yu. Rubina, E.I. Dementieva, A.A. Stomakhin, E.L. Darii, S.V. Pan'kov, V.E. Barsky, S.M. Ivanov, E.V. Konovalova, A.D. Mirzabekov, Hydrogel-based protein microchips: manufacturing, properties, and applications, Bio Techniques, 2003, 34,1008-1022.
   In the proposed invention microchips based on three-dimensional hydrogels are used for developing a method for quantitative determination of biological toxins.

### Disadvantages

A disadvantage of conventional immunoassay methods is that they do not allow carrying out a simultaneous analysis of several compounds, particularly biotoxins, in a sample. Multiple parallel analysis of several compounds is achieved with the use of microchips.

Methods for analyzing biological toxins on microchips, described in the literature, are disadvantageous in that they involve a complicated technology of manufacturing microchips: combining several blocks, providing channels for feeding solutions, connecting of electrodes. Signals are recorded with the use of complicated and costly equipment: a confocal microscope, Raman spectroscopy, etc. These disadvantages have been overcome in developing a method for the immunoassay of biological toxins with the use of three-dimensional hydrogel-based microchips.

### Disclosure of the Invention

It is an object of the invention to provide a method for quantitative detection of biological toxins of bacterial, plant and animal origin, which allows carrying out simultaneous parallel analysis of several biotoxins in a sample with a sensitivity not inferior to or even superior to the sensitivity of standard immunological tests. Said object is accomplished by the provision of a method for quantitative detection biological toxins using by-drogel-based microchips. Hydrogel elements having a diameter of 100 to 600 µm and a height of 30 to 100 µm containing immobilized antibodies against biotoxins or biotoxins are prepared by a method of photo- or chemically induced polymerization and are covalently attached to the surface of a support (glass, plastic or silicon). The proposed method comprises the following steps:
*a)* manufacturing a biological microchip comprising an ordered array of three-dimensional hydrogel cells on a solid support, containing immobilized antibodies to various biotoxins, or biotoxins, wherein each separate cell contains an immobilized antibody to an individual biotoxin or an individual biotoxin;
*b)* incubating a microchip in a reaction medium comprising a sample containing biotoxins to be analyzed to form immune biotoxin-antibody complexes, said incubating being carried out, if necessary, under stirring conditions;
*c)* detecting the formed complex;
*d)* quantitative detection of the biotoxin being analyzed.

When a reaction medium containing a sample to be analyzed (a biotoxin) is applied to a microchip, the formation of immune complexes with specific ligands immobilized in the gel cells of the microchip takes place. Detection of the formed complex in step *c)* and subsequent quantitative detection in step *d)* are carried out in the format of a direct, competitive or sandwich immunoassay.

For a direct immunoassay, the reaction medium in step *b)* contains a biotoxin to be analyzed, which forms a specific complex with the chip-immobilized antibodies against this toxin.

A competitive analysis is carried by using a microchip with immobilized biotoxins or immobilized antibodies. In the first case the reaction medium in step *b)* additionally contains labeled antibodies to the biotoxin to be analyzed, and there takes place a competition of labeled antibodies for binding to the biotoxin in solution and to the biotoxin immobilized on the microchip. In another variant of the competitive analysis the microchip contains immobilized antibodies, and the reaction medium in step *b)* additionally contains a labeled biotoxin. A competition between the labeled and non-labeled biotoxin for binding to the immobilized antibodies takes place.

In the case of a sandwich immunoassay, the microchip contains immobilized antibodies, the reaction medium in step *b)* contains the biotoxin to be analyzed, which forms a specific complex with the antibodies immobilized on the chip. After the formation of said complex, the microchip is developed with labeled antibodies specific to another epitope of the given biotoxin.

When necessary, step *b)* is carried out under stirring conditions, which considerably reduce the analysis time.

The methods of direct and competitive immunoassay can be carried out both for toxins of protein nature and for low-molecular toxins. A sandwich variant of immunoassay can be carried out only for biotoxins of protein nature, for which antibodies specific to different protein molecule epitopes can be obtained.

The immunoassay results are recorded with the aid of fluorescence, chemiluminescence, or mass spectrometry directly from the gel elements of the microchip. Antibodies or biotoxins may contain fluorescent labels or may be conjugates with proteins or other compounds capable of participating in reactions leading to the emission of light (chemi- or bioluminescence). Mass-spectral detection does not require introducing additional labels.

Quantitative biotoxin detection is effected by carrying out steps *a) - c)* with standard (reference) samples containing known concentrations of the biotoxin to be analyzed. A calibration curve "signal from microchip gel cell element vs. biotoxin concentration" is plotted. After that steps *a)* - *c)* are carried out with the sample to be analyzed, and the concentration of the biotoxin to be analyzed in the sample is determined from the calibration curve. The proposed method allows carrying out a simultaneous parallel analysis of several biotoxins in a sample. A microchip for parallel multiple analysis contains immobilized antibodies against several toxins and/or several immobilized biotoxins. In each separate gel element an antibody to an individual biotoxin or an individual biotoxin is immobilized. After incubating the microchip in a reaction medium comprising a sample containing several biotoxins to be analyzed, specific immune biotoxin-antibody complexes are formed. In the case of direct immunoassay, the reaction medium contains a sample comprising several toxins to be analyzed, and after incubating the microchip with the sample signals are recorded from the microchip elements containing a corresponding immobilized antibody. For competitive analysis, the reaction medium in step *b)* additionally contains a mixture of labeled antibodies to all the biotoxins to be analyzed or a mixture of all the labeled biotoxins to be analyzed. In the case of sandwich immunoassay, the development of the microchip after its incubation in the reaction medium comprising a sample is effected with a mixture of labeled antibodies against all the biotoxins to be analyzed.

The sensitivity of the proposed method for detection of biological toxins is not inferior to the sensitivity of the plate immunoassay variant. For instance, the limit of ricin detection by sandwich immunoassay with the use of gel microchips was 0.1 ng/ml (Table 1).

The proposed method for the analysis biotoxins, based on using gel microchips offers a number of essential advantages over microchips described in the literature: a developed three-dimensional gel structure has much larger capacity for immobilization as compared with two-dimensional microchips, whereby the analysis sensitivity is greatly enhanced; in addition, immobilized proteins are in a hydrophilic environment, and there is no contact with the hydrophilic surface of the support, this contributing to preserving the biological activity of the immobilized molecules and providing high stability under storage.

### Brief Description of the Figures

The proposed invention is illustrated by the following Figures, in which:

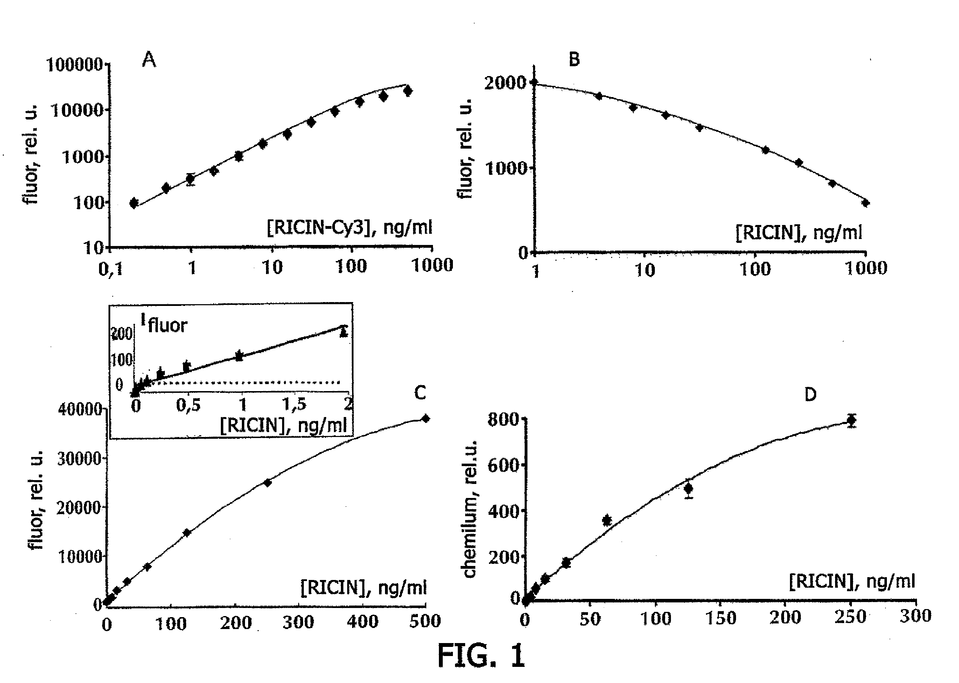

**Figure 1** demonstrates the results of ricin immunoassay on microchips.
   **A.** Direct immunoassay. Microchips contained gel elements with immobilized antibodies against ricin Rch1, 0.1 mg/ml. Dependence of fluorescence intensity on Cy3-labeled ricin concentration in solution.
   **B.** Competitive analysis. Microchips contained gel elements with immobilized ricin, 0.05 mg/ml. Dependence of fluorescence intensity on ricin concentration in solution after ricin incubation with solution of Cy3-labeled antibodies 1RK2, 4 µg/ml.
   **C.** Sandwich immunoassay with fluorescent recording. Microchips contained gel elements with immobilized antibodies against ricin Rch1, 0.1 mg/ml; microchips after incubation with ricin solutions were developed with Cy3-labeled antibodies against ricin 1RK1, 20 µg/ml. Dependence of fluorescence intensity on ricin concentration in solution. The inset in Fig. 1C shows fluorescent signals at low ricin concentrations. The dotted line corresponds to the fluorescence intensity 3 times higher than the background signal spread; the limit of ricin detection was 0.1 ng/ml.
   **D.** Sandwich immunoassay with chemiluminescent recording. Microchips contained gel elements with immobilized antibodies against ricin 1RK2, 0.1 mg/ml; the microchips, after incubating with ricin solutions, were developed with biotinylated antibodies against ricin 2RK1 (40µg/ml), avidin-peroxidase conjugate (45 µg/ml) and chemiluminescent peroxidase substrates. Dependence of chemi luminescence intensity on ricin concentration in solution.
      Each plotted point is an average of four parallel measurements.

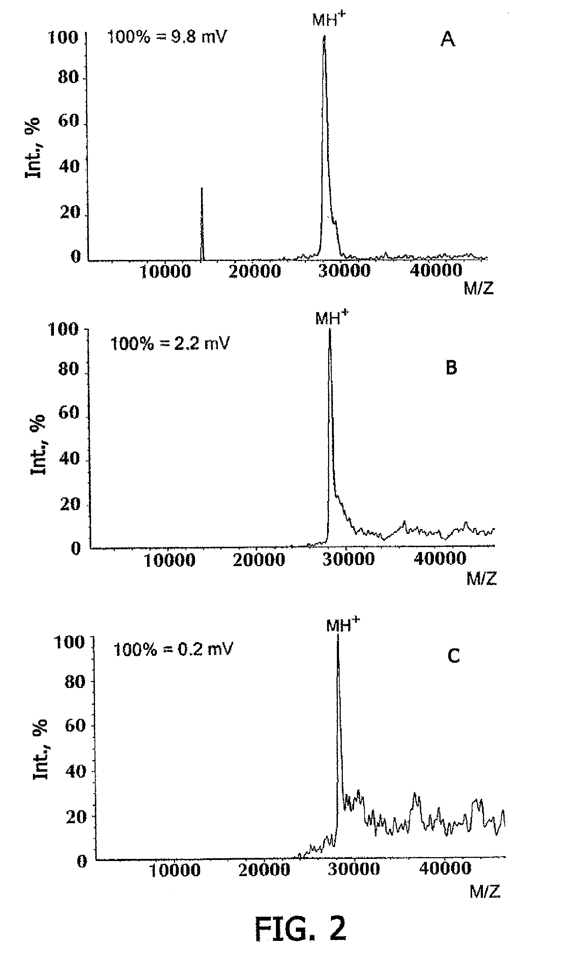

**Figure 2** demonstrates MALDI-TOF mass spectra of staphylococcal enterotoxin B, obtained from gel elements of a microchip with immobilized monoclonal antibodies against this toxin S222, 0.1mg/ml, after incubating the microchip with the toxin solution. Biotoxin concentrations in solution are 100 (A), 10 (B) and 1 (C) µg/ml.

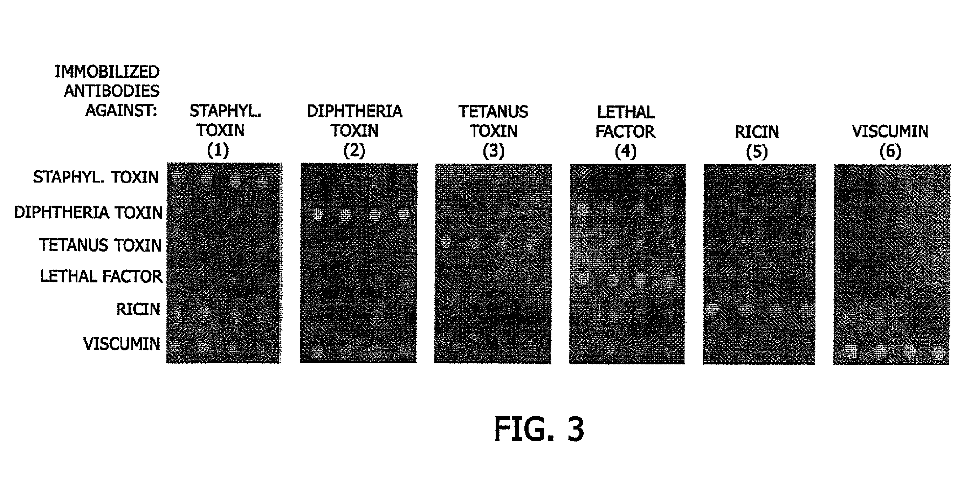

**Figure 3** shows the results of a simultaneous analysis of several biotoxins on one microchip. Microchips contained gel elements with immobilized monoclonal antibodies against staphylococcal toxin (S222), diphtheria toxin (7D9), tetanus toxin (3D2C6), lethal factor of anthrax toxin (10EDG7), ricin (1RK1) and viscumin (TAS). Each antibody was immobilized in 4 gel elements in a concentration of 0.1 mg/ml. Fluorescent images were obtained after incubating the microchip with a solution of staphylococcal toxin (chip 1), diphtheria toxin (chip 2), tetanus toxin (chip3), lethal factor of anthrax toxin (chip 4), ricin (chip 5) and viscumin (chip 6) and developing with a mixture of Cy3-labeled monoclonal antibodies against all the 6 biotoxins under investigation (antibodies against staphylococcal enterotoxin B S643, antibodies against diphtheria toxin 2A3, antibodies against tetanus toxin 3D10B11, antibodies against lethal factor of anthrax toxin 3B4D9, antibodies against ricin Rch1, antibodies against viscumin MNA9). The concentration of biotoxins in solution was 50 ng/ml (for each of the toxins), the concentration of each developing antibody in the mixture was 20 µg/ml, the total concentration of the labeled antibodies in the mixture was 120 µg/ml.

### Embodiment of the Invention

Hydrogel microchips for quantitative detection of biological toxins are manufactured by a method of photo- or chemically induced radical polymerization according to the patented technology [9]. A microchip comprises an ordered array of three-dimensional hydrogel cells on a solid support, which contain immobilized antibodies to various bacterial, plant or animal biotoxins, or immobilized biotoxins of various nature. In each separate cell of the microchip an individual ligand is immobilized. Binding of a ligand to a hydrogel matrix can be effected either directly during its immobilization in the process of gel formation or via the formation of specific avidin (streptavidin)-biotin complexes, such as immobilized avidin-biotinylated antibody or nitrilotriacetic acid-recombinant protein containing a polyhistidine fragment, and others.

Antibodies of various types, all types of immunoglobulins (IgG, IgM, IgA, IgD, IgE), monoclonal antibodies, polyclonal antibodies, recombinant antibodies, various types of mini-antibodies, including Fab-fragments and single-chain antibodies (scFv and others), as well as low-molecular biotoxins and biotoxins of protein nature are used as the ligands. The ligands for the formation of a specific complex with biotoxins to be analyzed can also be aptamers - DNA or RNA molecules capable of high-affinity interaction with a corresponding compound. For example, an RNA-aptamer consisting of 31 nucleotides, specific to ricin A-chain bacterial toxins, phytotoxins and zootoxins [13] and DNA aptamers specific to cholera toxin and to staphylococcal enterotoxin B [14] have been obtained.
[13] J.R. Hesselberth, D. Miller, J. Robertus, A.D. Ellington, In vitro selection of RNA molecules that inhibit the activity of ricin A-chain, J. Biol. Chem., 2000, 275, 4937-4942.
[14] J.G. Bruno, J.L. Kiel, Use of magnetic beads in selection and detection of biotoxin aptamers by electrochemiluminescence and enzymatic methods, Biotechniques, 2002, 32, 178-183.

The method of polymerization immobilization, employed for manufacturing of microchips, allows the obtaining of microchips containing immobilized DNAs, RNAs and proteins [9-12].

The type of compounds employed for the immobilization depends on the object to be analyzed and on the method of analysis (direct, competitive, sandwich immunoassay, and others).

The method for carrying out quantitative detection of biotoxins with the use of hydrogel microchips comprises the following steps:
*a)* manufacturing a biological microchip comprising an ordered array of three-dimensional hydrogel elements on a solid support, which are obtained by a method of photo- or chemically induced polymerization and contain immobilized antibodies to various bacterial, plant or animal biotoxins, or biotoxins, wherein each separate element contains an immobilized antibody to an individual biotoxin, or an individual biotoxin;
*b)* incubating a microchip in a reaction medium comprising a sample containing biotoxins to be analyzed to form immune biotoxin-antibody complexes, said incubating being carried out, if necessary, under stirring conditions;
*c)* detecting the formed complex;
*d)* quantitative detection of the biotoxin being analyzed.

The reaction medium used for carrying out analysis comprises buffer solutions conventionally employed in immunoassay, for example, a 0.01 M phosphate buffer, pH 7.2, containing 0.15 M NaCl, 0.05 M tris-HCl, pH 7.4, etc. For reducing nonspecific interactions, polyvinyl alcohol, bovine serum albumin and sucrose, dry milk proteins, and other so-called "blocking buffers" well known to those skilled in the art, are added to the reaction medium.

The method for quantitative detection of biotoxins can be effected as various types of immunoassay, for instance, as direct, competitive, and sandwich immunoassay (Example 1). For direct immunoassay, the microchip contains immobilized antibodies against the biotoxin to be analyzed, and the reaction medium in step *b)* contains the biotoxin to be analyzed, which forms a specific immune complex with the immobilized antibodies. In the case of fluorescent recording the biotoxin to be analyzed contains a fluorescent label, in the case of chemiluminescent recording the biotoxin is a conjugate with a protein or other compound capable of participating in reactions leading to the emission of light (chemi- or bioluminescence). The fluorescent or chemiluminescent signal being recorded from the microchip elements with the immobilized antibodies is proportional to the biotoxin concentration (Example 1, Fig. 1A). Methods of introducing a fluorescent label or of preparing conjugates with a protein or other compound are well known in this field of the art. In particular, procedures described in G.T. Hermanson, Bioconjugate Techniques, Academic Press, San Diego, 1996, can be used. In the case of mass-spectral detection, introducing an additional label is not required.

Competitive immunoassay is carried out using a microchip with immobilized biotoxins or immobilized antibodies. In a first variant the reaction medium in step *b)* additionally contains fluorescently labeled antibodies to the biotoxin to be analyzed (or corresponding antibody conjugates). The reaction mixture, prior to applying it to the microchip, is incubated with a standard sample or with the sample to be analyzed. During the incubation of the microchip with the reaction mixture there takes place competition of the labeled antibodies for binding to the biotoxin in solution and to the biotoxin immobilized on the microchip. The fluorescent or chemiluminescent signal recorded from the microchip elements with the immobilized biotoxins is the higher the smaller is the concentration of the toxin being analyzed in the sample (Example 1, Fig. 1B).

In the other variant of the competitive immunoassay the microchip contains immobilized antibodies, and the reaction mixture in step *b)* additionally contains a fluorescently labeled biotoxin (or a corresponding biotoxin conjugate). A competition takes place between the labeled and non-labeled biotoxin for binding to the immobilized antibodies. The fluorescent or chemiluminescent signal recorded from the microchip elements with the immobilized antibodies is the higher the smaller is the concentration of the toxin being analyzed in the sample.

In the case of sandwich immunoassay the microchip contains immobilized antibodies, the reaction medium in step *b)* contains the biotoxin to be analyzed, which forms a specific complex with the antibodies immobilized on the chip. After the formation of said complex, the microchip is developed with fluorescently labeled antibodies (or with corresponding antibody conjugates) specific to another epitope of the given biotoxin. The fluorescent or chemiluminescent signal being recorded from the microchip cells with the immobilized antibodies is proportional to the biotoxin concentration (Example 1, Figs. 1C, D).

Direct and competitive immunoassays on microchips can be carried out both for toxins of protein nature and low-molecular toxins. Sandwich immunoassay variant is possible, as a rule, only for biotoxins of protein nature, for which antibodies can be obtained, that are specific to different epitopes of the protein molecule (immobilized binding antibodies and developing labeled antibodies).

Quantitative detection of biotoxins is effected by carrying out steps *a)-c)* with known concentrations of the biotoxin to be analyzed and plotting a calibration curve from which the amount of biotoxin to be analyzed in the sample is determined. For all variants of immunoassay a calibration chart is plotted, which shows the dependence of the intensity of fluorescence or chemiluminescence signal from corresponding gel elements of the microchip on the known biotoxin concentrations in a standard (reference) solution. The unknown biotoxin concentration in the sample is determined from the calibration curve, using the intensity of the signal obtained after the interaction of the sample being analyzed with the microchip. The biotoxin detection limit is determined as the concentration corresponding to the intensity of a fluorescence/chemiluminescence signal 3 times higher than the background signal spread.

The results of the immunoassay of biotoxins using gel microchips can be recorded by several methods: by fluorescence intensity; by the chemiluminescence intensity; by mass spectrometry directly from the gel elements of the microchip.

When the results are recorded by fluorescence intensity, antibodies against biotoxins and biotoxins labeled with fluorescent dyes are used, the dyes are Texas red, tetramethyl rhodamine, fluorescein, cyanine 3 and 5 (Cy3, Cy5), but the range is not limited thereby. The fluorescent signals from the microchip elements are recorded with the help of fluorescence microscopes or scanning microscopes of various types which allow recording signals with the fluorescent label employed.

When recording the results by chemiluminescence intensity, conjugates of antibodies, biotoxins or avidin (for biotinylated proteins) are used, for example, conjugates with such enzymes as horseradish peroxidase, alkaline phosphatase, β-galactosidase, luciferase, the range being not limited thereby, which yield chemiluminescent signals as the result of corresponding enzymatic reactions, for instance, of peroxidase-catalyzed luminol oxidation with hydrogen peroxide. The chemiluminescent signals are recorded with the help of CCD-cameras or other recording devices, particularly with the help of fluorescence microscopes, the excitation light source being switched off.

For recording the results with the help of MALDI-TOF mass spectrometry, a procedure has been developed for obtaining MALDI-TOF mass spectra directly from the microchip gel elements. In this case the procedure of recording the results of analysis comprises: treating the microchip with a solution which destroys the complex formed by the biotoxin under investigation with the antibody against this toxin, immobilized on the chip (elution of biotoxin from the microchip), mass spectrometric analysis directly from the gel element, and identification of the biotoxin by molecular weight (Example 2, Fig. 2).

Example 3 shows the results of immunoassay of 6 different biological toxins on hydrogel microchips. The method for quantitative detection of biotoxins with the use of hydrogel microchips is characterized by high sensitivity, not inferior to the sensitivity of conventional immunological methods: for ricin the detection limit was 0.1 ng/ml.

The proposed method for quantitative detection biotoxins using hydrogel microchips allows carrying out simultaneous, parallel analysis of samples for the presence of several toxins, whereby the efficiency of detecting is sharply enhanced and the amount of the material to be investigated is considerably reduced: 20 µl of a sample to be investigated are sufficient for analysis. The microchip for parallel multiple analysis contains immobilized antibodies against several toxins and/or several immobilized biotoxins. In the case of direct immunoassay the reaction medium contains a sample comprising one or several toxins to be analyzed, and, after incubating the microchip with the sample, signals from the chip elements containing corresponding immobilized antibodies are recorded. For competitive immunoassay the reaction medium the reaction medium in step *b)* additionally contains a mixture of labeled antibodies to all biotoxins being analyzed or a mixture of all labeled biotoxins being analyzed. Carrying out simultaneous sandwich immunoassay of 6 different biological toxins on one microchip with developing the microchip with a mixture of 6 fluorescence-labeled antibodies is demonstrated in Example 4. The sensitivity of parallel analysis proved to be no less than the sensitivity for identifying one toxin.

When necessary, the step of microchip incubation in a reaction medium comprising a sample containing biotoxins to be analyzed (step *b*) is carried out under stirring which considerably reduces the analysis time (Example 5). Stirring can be carried out, for instance, by switching the direction of the reaction solution flow from direct to reverse with the help of pumps of various types, by ultrasound, by electrophoresis, but the range of suitable methods is not limited to those indicated here.

An advantage offered by hydrogel microchips over two-dimensional chips described in the literature is the hydrophilic environment of molecules immobilized in the gel and the absence of contact with the hydrophobic surface of the support, this being of particular importance for protein molecules. The hydrophilic environment contributes to preserving the biological activity of proteins and enzymes and provides their stabilization during storage. Microchips with immobilized antibodies are stable during at least half a year when stored in the presence of glycerin at 10°C (Example 6).

Further the invention is illustrated by particular embodiments which are presented for illustrative purposes only. It is not presumed that the presented examples are exhaustive or limit the invention to particular disclosed forma, and it is obvious that a large number of modifications and versions are possible without departing from the spirit of the invention and without going beyond the scope of the set of claims.

### Example 1. Quantitative immunoassay of ricin using hydrogel microchips

Hydrogel microchips containing immobilized antibodies against ricin and ricin were obtained by following the earlier patented polymerization immobilization technology [9]. For reducing non-specific interactions in all variants of analysis, microchips were preincubated in a 0.01 M phosphate buffer, pH 7.2, containing 0.15 M NaCl, 1% polyvinyl alcohol (PVA-50) or 3% bovine serum albumin (BSA) and 4% sucrose ("blocking buffer"), for 2 h at room temperature. Before carrying out the analysis, ricin solutions (sample solutions) were diluted with 0.01 M phosphate buffer, pH 7.2, containing 0.15 M NaCl, 0.15% PVA-50 and 0.15% polyvinyl pyrrolidone 360 (PVP-360).

Direct immunoassay. To microchips containing gel elements with immobilized monoclonal antibodies against ricin Rch1 and other biotoxins (antibodies against staphylococcal enterotoxin B S222, antibodies against tetanus toxin 3D2C6, antibodies against diphtheria toxin 7D9, for controlling cross-couplings (concentration of antibodies immobilized in gel, 0.1 mg/ml), 20µl of Cy3-labeled ricin solutions were added, and kept overnight at 10°C. After washing the microchips with 0.01 M phosphate buffer, pH 7.2, 0.15 M NaCl, 0.1% Tween-20 (15 min., room temperature), the intensity of fluorescent signals of the gel elements of the microchips was measured.

For competitive immunoassay, microchip with immobilized ricin (0.1 mg/ml) was used. The solution containing ricin to be detected was incubated with a solution of Cy3-labeled monoclonal antibodies against ricin 1RK2 (4 µg/ml) for 2-4 h at 37°C with stirring. The mixture (20 µl) after incubation was applied to the microchip and kept for 2 hours at 37°C. After washing (0.01 M phosphate buffer, pH 7.2, 0.15 M NaCl, 0.1% Tween-20, 15 min, room temperature), the intensity of fluorescent signals was measured.

In the sandwich variant of immunoassay microchips with immobilized monoclonal antibodies against ricin 1RK2 and other biotoxins were manufactured (antibodies against staphylococcal enterotoxin B S222, antibodies against tetanus 3D2C6, antibodies against 7D9 for the control of cross-couplings (concentration of antibodies immobilized in gel, 0.1 mg/ml), there was added 20µl of Cy3-labeled ricin solution, and the chips were incubated overnight at 10°C. After short-time (15 min) washing with 0.01 M phosphate buffer, pH 7.2, 0.15 M NaCl, 0.1 % Tween-20, the microchips were developed with Cy3-labeled antibodies 1RK1 specific to an other antigen epitope, 20 µg/ml (fluorescent detection), 40 min, room temperature. After washing (0.01 M phosphate buffer, pH 7.2, 0.15 M NaCl, 0.1 % Tween-20, 125 min, room temperature) the intensity of fluorescent signals was measured. In the case of chemiluminescent detection, to the microchip, after the interaction with ricin, 20 µl of biotinylated antibodies against ricin 2RK1 with the concentration of 40 µg/ml, and then 20 µl of avidin conjugate with horseradish peroxidase with the concentration of 45 µg/ml and chemiluminescent substrates of peroxidase (luminol, H₂O₂) were applied, the luminescent signal was detected during 60sec.

The Cy3-labeled antibodies against ricin and ricin, as well as the biotinylated antibodies and the avidin-peroxidase conjugate, were obtained by following the known procedures, described, e.g., in [15].
[15] G.T. Hermanson, Bioconjugate Techniques, Academic Press, San Diego, 1996.

For measuring fluorescent signals a fluorescence microscope developed at the laboratory of biochips of the 1MB RAS [16] was used, the microscope is equipped with a CCD camera and provided with computer programs for visualizing images and treating obtained data.
[16] V. Barsky, A. Perov, S. Tokalov, A. Chudinov, E. Kreundin, A. Sharonov, E. Kotova, A. Mirzabekov, Fluorescence data analysis on gel-based biochips, J. Biomol. Screening, 2002, 7, 247-257.

The microscope allows one to simultaneously analyze data from all the microchip elements and obtain two-dimensional and three-dimensional images of the fluorescent signal from the gel elements. For measuring chemiluminescent signals, the same microscope was used, with the excitation source switched off.

For all variants of the immunoassay, dependence curves of the fluorescence or chemiluminescence intensity vs. ricin concentration in solution were plotted (Fig. 1). The limit of ricin detection was calculated as the concentration corresponding to the signal intensity 3 times that of the background signal spread.

For direct immunoassay of ricin, the dependence of the fluorescence intensity of the microchip gel elements on the concentration of Cy3-labeled ricin in the concentration range of 0.2-500 ng/ml was observed; linear dependence was observed in the concentration range of 0.2-60 ng/ml (Fig. 1A), the detection limit was 0.2 ng/ml.

In the case of competitive immunoassay, the fluorescence intensity of the microchip gel elements was inversely proportional to the ricin concentration in solution (Fig. 1B), and the detection limit was 4 ng/ml.

Calibration charts for the sandwich immunoassay of ricin with the fluorescent and chemiluminescent detection are shown in Fig. 1C and D. The dependence of the fluorescence and chemiluminescence intensity of the microchip gel elements on the ricin concentration was observed in the ricin concentration range of 0.1-500 ng/ml (fluorescent detection) and 0.7-500 ng/ml (chemiluminescent detection). The inset in Fig. 1C illustrates the detection limit determination: the dashed line corresponds to the fluorescence intensity 3 times that of the background signal spread. Hence, the minimum ricin concentration reliably determined by the given method is 0.1 ng/ml. For the immunoenzymatic assay of ricin on the microchip with chemiluminescent detection the detection limit was 0.7 ng/ml.

Under these analysis conditions, particularly with the employed by us procedures of blocking and washing microchips, non-specific signals, i.e., signals from the microchip cells containing antibodies to other toxins, did not differ from the background signals or from signals of empty gel elements.

### Example 2. Identification of proteins on microchip gel element by direct mass-spectroscopic analysis. Direct analysis of staphylococcal enterotoxin B on microchip with mass-spectroscopic recording

Microchip has been manufactured, containing in gel elements immobilized monoclonal antibodies to staphylococcal enterotoxin B S222 (0.1 µg antibodies/gel element). After polymerization, the microchip was washed with 0.01 M phosphate buffer containing 0.15 M NaCl, 0.1% Tween-20, with stirring for 1 hour (20°C). The microchip was incubated with a solution of staphylococcal enterotoxin in 0.01 M phosphate buffer, pH 7.2, containing 0.15 M NaCl (20 hrs, 20 °C), and further washed to remove the protein non-specifically bound to the gel, first by treating with 0.01 M phosphate buffer, pH 7.2, containing 0.15 M NaCl, 0.1% Tween-20 (2 hours with stirring, 20 °C), then with water. Before carrying mass-spectroscopic analysis, the antigen-antibody complex was destroyed and the antigen was eluted to the gel surface by adding to each microchip cell 1 µl of saturated solution of a matrix for MALDI monitoring (sinapinic acid) in solution of 10% formic acid in 30% aqueous acetonitrile. The microchip was kept for 20 min at room temperature in a moist chamber, then dried on a heating table at 40°C.

Mass spectra obtained directly from the gel elements of the microchip, after the interaction with staphylococcal toxin solutions of different concentrations, are shown in Fig. 2. Staphylococcal enterotoxin B was identified by its molecular mass equal to 28400 Da. As is seen from the pattern, a quantitative correlation can be observed between the absolute intensity of molecular ion peaks and the enterotoxin concentration in the concentration range of 1-100 µg/ml. Mass spectra from the gel elements without antibodies to this toxin, but incubated with the antigen solution, did not contain the abovementioned peaks.

### Example 3. Quantitative immunoassay of various biological toxins, using hydrogel microchips

The results of quantitative immunoassay of various biological toxins on gel microchips manufactured by the method of polymerization immobilization, are shown in Table 1. Besides the immunoassay of ricin, described in Example 1, immunoassay of viscumin, staphylococcal enterotoxin B, tetanus toxin, diphtheria toxin, and lethal factor of anthrax toxin was carried out. Direct, competitive and sandwich immunoassay with fluorescent and chemiluminescent recording was carried out by following the procedures described in Example 1, using the antibodies indicated in Table 1. Table 1 shows also the range of concentrations at which the dependence of the intensity of the fluorescent or chemiluminescent signal of the microchip gel cells on the biotoxin concentration was observed; the lower limit of the range corresponds to the detection limit calculated as described in Example 1.

### Example 4. Simultaneous immunoassay of several biotoxins on one microchip

The main advantage of microchips over conventional immunoassay methods is the possibility of quantitative assay of samples simultaneously for the presence of several antigens. Microchips with gel elements have been manufactured, wherein the gel elements contained immobilized antibodies to 6 biotoxins (ricin, viscumin, staphylococcal enterotoxin B, tetanus toxin, diphtheria toxin, lethal factor of anthrax toxin). After incubating the microchip with a solution containing one of the toxins being investigated, the microchip was developed by a mixture of Cy3-labeled secondary antibodies against all the 6 toxins (Fig. 3). Pairs of antibodies for parallel analysis of several toxins were selected in such a manner that the non-specific interaction with other toxins and antibodies were minimized. For instance, in the case of parallel analysis, antibodies against ricin 1RK1 were used as immobilized antibodies, and antibodies Rch1 were used as developing ones, but not vice versa, as in Example 1 for the sandwich immunoassay, because the immobilized antibodies Rch1 give non-specific signals with the Cy3-labeled antibodies against the staphylococcal enterotoxin B SEB 643. Bright fluorescent signals were observed in the gel elements containing corresponding antibodies. The biotoxin detection limits for the parallel analysis proved to be the same as in detecting each toxin separately (Table 1).

### Example 5. Acceleration of biotoxin immunoassay when stirring the sample under analysis

Sandwich-immunoassay of ricin was carried out as described in Example 1, but microchips were incubated with ricin solutions during 1 hour with stirring. Stirring was performed with the aid of a peristaltic pump. For this purpose the microchip was placed into a flow-type chamber having a volume of 50 µl, provided with pipes for feeding the sample. The chamber was connected to a peristaltic pump, and 100 µl of ricin solution were placed into the system. The sample was stirred by switching over the direction of flow from direct to reverse every 2 sec.; the flow rate was 1.5 ml/min. The analysis with stirring was carried out simultaneously on 6 microchips, i.e., measuring of 6 samples with different ricin concentrations was carried out. After incubation, the microchips were developed with Cy3-labeled secondary antibodies against ricin. The calibration chart for detecting ricin by the sandwich-immunoassay with stirring for 1 hour was analogous to the calibration chart when carrying out sandwich-immunoassay with incubation overnight without stirring (Fig. 1C). Thus, stirring the sample made it possible to shorten essentially the immunoassay time with the use of microchips.

### Example 6. Stability of hydrogel microchips with immobilized antibodies

Microchips with immobilized antibodies against ricin were manufactured as described in Example 1. The microchips were stored in a moist chamber at 10°C in the presence of 20% glycerin. Sandwich immunoassay of ricin with fluorescent recording was carried out, a calibration curve "fluorescent signal intensity vs. ricin concentration in solution" was plotted, and the ricin detection limit was determined as described in Example 1. The ricin detection limit on the microchips after 6 months of storage proved to be 0.1 ng/ml, i.e., the same as for the microchips directly after their manufacture. Thus, microchips with immobilized antibodies fully preserve their activity during at least 6 months of storage.

**Table 1. Results of quantitative immunoassay of various biotoxins on a microchip**

| Biotoxin | Type of analysis (signal recording) | Antibodies | Range of concentrations for analysis on microchips, ng/ml | Detection limit for standard test systems, ng/ml |
|---|---|---|---|---|
| Ricin | Direct immunoassay (fluorescence) | Rch1 (immobilized) | 0.2-500 | 0.1 |
| | Competitive immunoassay (fluorescence) | 1RK2-Cy3 | 4-1000 | |
| | Sandwich-immunoassay (fluorescence) | Rch1 (immobilized), 1RK1-Cy3 (developing) | 0.1-500 | |
| | Sandwich-immunoassay (chemiluminescence) | 1RK2 (immobilized), 2RK1-biotin+svidin-IIX (developing) | 0.7-500 | |
| Viscumin | Sandwich-immunoassay (fluorescence) | TAS (immobilized), MNA9-Cy3 (developing) | 2-1000 | 0.08-5.0 |
| Staphylococcal enterotoxin B | Direct immunoassay (fluorescence) | S222 (immobilized) | 50-20000 | 0.01-1.0 |
| | Sandwich-immunoassay (fluorescence) | S222 (immobilized), S643-Cy3 (developing) | 1-300 | |
| Tetanus toxin | Sandwich-immunoassay (fluorescence) | 3D2C6 (immobilized), 3D10B11-Cy3 (developing) | 10-1000 | 100 |
| Diphtheria toxin | Direct immunoassay (fluorescence) | 2A3 (immobilized) | 12-25000 | 0.1-5.0 |
| | Sandwich-immunoassay (fluorescence) | 4C7 (immobilized), 2A3-Cy3 (developing) | 1-500 | |
| Lethal factor of anthrax toxin | Direct immunoassay (fluorescence) | 8D5B 11 (immobilized) | 20-20000 | 500 |
| | Sandwich-immunoassay (fluorescence) | 10EDG7 (immobilized), 3B4D9-Cy3 (developing) | 4-4000 | |
| | Sandwich-immunoassay (chemiluminescence) | 10EDG7 (immobilized), 3B4D9-biotin+avidin-IIX (developing) | 6-1000 | |

## Claims

1. A method for quantitative detection of biotoxins in a sample, comprising the steps of:
*a)* manufacturing a biological microchip comprising an ordered array of three-dimensional hydrogel elements on a solid support, obtained by a method of photo- or chemically induced polymerization and containing immobilized antibodies to various bacterial, plant or animal biotoxins, or immobilized biotoxins, wherein an antibody to an individual biotoxin or an individual biotoxin is immobilized in each separate cell;
*b)* incubating the microchip in a reaction medium which comprises a sample containing biotoxins to be analyzed, for forming immune biotoxin-antibody complexes, which incubation, when necessary, is carried out under stirring conditions;
*c)* detecting the formed complex;
*d)* quantitative detection of the biotoxin being analyzed.

2. The method as claimed in claim 1, wherein the immobilized antibodies comprise antibodies selected from the group of antibodies to ricin, viscumin, staphylococcal enterotoxin B, tetanus toxin, diphtheria toxin, lethal factor of anthrax toxin.

3. The method as claimed in claim 1, wherein the immobilized biotoxins comprise biotoxins selected from the group comprising ricin, viscumin, staphylococcal enterotoxin B, tetanus toxin, diphtheria toxin, lethal factor of anthrax toxin.

4. The method as claimed in claim 1, wherein detecting the complex formed in step *c)* and subsequent quantitative detection in step *d)* are carried out in a format of direct immunoassay.

5. The method as claimed in claim 1, wherein the reaction medium in step *b)* additionally contains antibodies to a biotoxin, and the detection of the complex formed between the biotoxin immobilized on the chip and the antibody against this biotoxin in step *c)* and subsequent quantitative detection in step *d)* are carried out in the format of competitive immunoassay.

6. The method as claimed in claim 1, wherein the reaction medium in step *b)* further contains a labeled biotoxin, and detection of the complex formed between the antibody immobilized on the chip and the biotoxin in step *c)* and subsequent quantitative detection in step *d)* are carried out in the format of competitive immunoassay.

7. The method as claimed in claim 1, wherein detection the complex formed in step *c)* and subsequent quantitative detection in step *d)* are carried out in the format of sandwich-immunoassay.

8. The method as claimed in claim 1, wherein quantitative detection of the biotoxin is effected by carrying out steps *a)-c)* with known concentrations of the biotoxin being analyzed and with plotting a calibration dependence curve, from which the amount of the biotoxin being analyzed in the sample is determined.

9. The method as claimed in claim 1, wherein in step *c)* detection of the formed complex is carried out fluorimetrically, chemiluminometrically or mass-spectrometrically.
